# DEMANDE DE BREVET EUROPEEN

(11) **EP 2 388 069 A1**
(43) Date de publication de la demande: **23.11.2011**
(21) Numéro de dépôt: 11290213.5
(22) Date de dépôt: 29.04.2011
(51) Int. Cl.: B01J 21/06, C07C 2/32, C07C 2/36, C07C 11/08

(54) **Procédé de dimérisation de l'éthylène en butène-1 utilisant une composition comprenant un complexe à base de titane et un ligand alcoxy fonctionnalisé par un hétéro-atome**

(30) Priorité: 18.05.2010 FR 1002089
(71) Demandeur: IFP Energies nouvelles, 92852 Rueil-Malmaison Cedex (FR)
(72) Inventeur: Grasset, Fabien, 69500 Bron (FR); Magna, Lionel, 69007 Lyon (FR)

(57) **Abrégé**

L'invention décrit un procédé de dimérisation sélective de l'éthylène en butène-1 mettant en oeuvre une composition catalytique comprenant au moins un complexe organométallique du titane ledit complexe organométallique contenant au moins un ligand de type alcoxy fonctionnalisé par un hétéro-atome choisi parmi l'azote, l'oxygène, le phosphore, le soufre, l'arsenic, l'antimoine ou par un groupement aromatique.

## Description

La présente invention concerne la dimérisation sélective de l'éthylène en butène-1. Un objet de l'invention est de fournir un procédé de dimérisation de l'éthylène utilisant une composition catalytique particulière.

### Art Antérieur:

Il est bien connu que les oléfines telles que l'éthylène, le propylène ou le butène-1 peuvent être dimérisées avec des systèmes catalytiques à base de métaux de transition tels que le nickel, le chrome, le titane, le zirconium ou d'autres métaux, en présence d'un cocatalyseur tel qu'un composé d'hydrocarbylaluminium, un halogénure d'hydrocarbylaluminium ou un aluminoxane.

Il a été décrit plusieurs types de ligands pour stabiliser l'espèce catalytique et orienter la sélectivité de la réaction de dimérisation. Dans le brevet U.S. 2.943.125, K. Ziegler a décrit une méthode de dimérisation de l'éthylène en butène-1 au moyen d'un catalyseur obtenu par le mélange de trialkylaluminium et d'un tetraalcoolate de titane ou de zirconium. Lors de la réaction se forme également une certaine quantité de polyéthylène de haute masse moléculaire qui gène considérablement la mise en oeuvre. Plusieurs améliorations ont été proposées pour diminuer le taux de polymère, en particulier dans le brevet U.S. 3.686.350 qui préconise l'emploi de composés organiques du phosphore, conjointement avec les éléments du catalyseur, dans le brevet U.S. 4.101.600 qui décrit le traitement du catalyseur par de l'hydrogène ou dans le brevet U.S. 3.879.485 qui décrit l'utilisation de divers éthers comme solvants du milieu réactionnel. Bien que ces modifications du système catalytique initial apportent une amélioration substantielle a la sélectivité de la réaction, elles se révèlent d'une utilisation peu pratique, en particulier dans un procédé industriel dans lequel il faut pouvoir séparer le butène-1 du solvant en laissant seulement des traces de composé polaire dans le butène. De ce point de vue, le brevet FR 2 552 079, a démontré que l'utilisation d'un éther en association avec un titanate d'alkyle en quantité voisine de la stoechiométrie et à un trihydrocarbylaluminium améliore appréciablement l'activité et la sélectivité des catalyseurs titanate d'alkyle-trihydrocarbylaluminium pour la dimérisation de l'éthylène en butène-1. Cet effet est plus marqué que celui qui est apporté par l'utilisation d'éthers en quantité correspondant à un usage comme solvant. Il présente également l'avantage d'éliminer l'emploi desdits éthers comme solvants, dont on a signalé les inconvénients.

Le principal inconvénient des systèmes catalytiques à base de titane conduisant à la formation sélective de butène-1 est la formation en quantité non négligeable de polymères. Cette formation de polymères peut être à l'origine d'une désactivation rapide du catalyseur ainsi que d'une difficulté considérable d'opérabilité sur une unité industrielle. Le contrôle de la quantité de polymères est donc un paramètre très important pour le devenir industriel de ce type de systèmes catalytiques.

Le contrôle de la co-production de polymères est dans la majeure partie des systèmes associé à l'utilisation d'additifs (organiques ou autres) ce qui très souvent complexifie la composition catalytique. Par ailleurs, s'ils sont performants pour contrôler la production de polyéthylène (PE), ces additifs conduisent souvent à des diminutions de productivité du catalyseur.

Un objectif de l'invention est de fournir une nouvelle composition catalytique pour la dimérisation sélective de l'éthylène en butène-1.

Un autre objectif de l'invention est de fournir un procédé de dimérisation sélective de l'éthylène en butène-1 mettant en oeuvre ladite composition catalytique, ledit procédé présentant une activité catalytique améliorée.

### Description de l'invention:

Il a maintenant été trouvé, qu'un procédé mettant en oeuvre une composition catalytique comprenant au moins un complexe organométallique du titane, ledit complexe organométallique contenant au moins un ligand de type alcoxy fonctionnalisé par un hétéroatome choisi parmi l'azote, l'oxygène, le phosphore, le soufre, l'arsenic et l'antimoine ou par un groupement aromatique, et ayant pour formule générale :

[Ti(OR)ₙ(Y)₍₄₋ₙ₎]

dans laquelle :
- Y est un radical hydrocarbyl comprenant de 1 à 30 atomes de carbone ou un radical choisi dans le groupe formé par les halogénures, les alcoxy R'O-, les amido R'₂N- et les carboxylates R'COO-, où R' est un radical hydrocarbyl, de préférence non fonctionnalisé, comprenant de 1 à 30 atomes de carbone,
- n peut prendre les valeurs entières de 1 à 4,
- Le ligand -OR est un composé organique choisi dans la famille des ligands alcoxy dont la structure générale est proposée ci-après :

   O-(CR¹⁰R¹¹)ₙ-X-L
dans laquelle :
- le groupement fonctionnel L est un groupement comprenant un hétéroatome choisi parmi l'azote, l'oxygène, le phosphore, le soufre, l'arsenic et l'antimoine ou un groupement aromatique,
- Le groupement X représente un groupement hydrocarboné (CR⁷R⁸), un atome d'oxygène, ou un groupement comprenant un atome d'azote -NR⁹,
- Les groupements R⁷, R⁸, R⁹, R¹⁰ et R¹¹ représentent un atome d'hydrogène ou une chaîne hydrocarbonée, cyclique ou non, comprenant 1 à 30 atomes de carbone, et comprenant optionnellement un hétéroatome.
- n peut prendre les valeurs entières de 0 à 30 et de préférence de 0 à 10, permettait d'obtenir une sélectivité très élevée pour la dimérisation sélective de l'éthylène en butène-1 et de limiter la formation de polymères.

Dans le cadre de l'invention, on définit le terme "alcoxy" comme étant un groupement répondant à la formule générale -OR dans laquelle le groupe R est un groupe alkyl ou alkyl substitué. Cette définition du terme "alcoxy" n'inclut pas les groupements de type aryloxy ou phenoxy. Dans la composition catalytique selon l'invention, le ligand de type alcoxy tel que défini ci dessus est fonctionnalisé par un hétéroatome choisi parmi l'azote, l'oxygène, le phosphore, le soufre, l'arsenic et l'antimoine ou par un groupement aromatique et répond à la formulation revendiquée.

De préférence, ledit groupement fonctionnel L est un groupement comprenant un hétéroatome, ledit groupement comprenant un hétéroatome étant choisi parmi les groupements -NR¹R², -OR³, -PR⁴R⁵, et -SR⁶ dans lesquels les groupements R¹, R², R³, R⁴, R⁵, R⁶ représentent un atome d'hydrogène ou une chaîne hydrocarbonée, cyclique ou non, comprenant 1 à 30 atomes de carbone.

De préférence, Y est un radical choisi dans le groupe formé par les alcoxy R'O- où R' est un radical hydrocarbyl, de préférence non fonctionnalisé, comprenant de 1 à 30 atomes de carbone. De manière également préférée, Y est un atome de chlore.

De préférence, le groupement (CR¹⁰R¹¹)ₙ est choisi parmi les groupements suivants : -CH₂-, -(CH₂)₂-, -(CH₂)₃-, -(CH₂)₄-, -(CH₂)₅-, -C(CH₃)₂-, -C(CH₃)₂-CH₂-, -C(CH₃)₂-CH₂-CH₂-, -C(CF₃)₂- , -C(CF₃)₂-CH₂- et -C(CF₃)₂-CH₂-CH₂-.

De préférence, ledit groupement fonctionnel L est choisi parmi les groupements suivants : méthoxy (-OMe), butoxy (-OBu), diméthylamino (-NMe₂), pyrrolidino (C₄H₈N), pyridino (-C₅H₄N), phosphino (-PR₂) dans lequel R est un groupement alkyl ou aryl substitué ou non, thiofène (-C₄H₃S), tétrahydrofurane (-C₄H₇O), furane (-C₄H₃O) et phényl (-C₆H₅), lesdits groupements pouvant être substitués ou non. Ledit groupement L est de préférence le groupement phosphino (-PR₂) dans lequel R est un groupement alkyl ou aryl substitué ou non.

De préférence, X représente un groupement hydrocarboné (CR⁷R⁸). De manière très préférée, X est un groupement hydrocarboné (CR⁷R⁸) choisi parmi les groupements -CH₂- et -C(CH₃)₂-.

La composition catalytique utilisée dans le procédé de dimérisation sélective de l'éthylène en butène-1 selon l'invention peut avantageusement également contenir un composé d'hydrocarbylaluminium, appelé agent activateur, choisi dans le groupe formé par les composés tris(hydrocarbyl)aluminium, les composés chlorés ou bromés d'hydrocarbylaluminium et les aluminoxanes.

Les tris(hydrocarbyl)aluminium et les composés chlorés ou bromés d'hydrocarbylaluminium répondent de préférence à la formule générale AlR"ₓZ₃₋ₓ dans laquelle R" représente un radical hydrocarboné monovalent contenant par exemple jusqu'à 12 atomes de carbone tel que alkyle, aryle, aralkyle, alkaryle ou cycloalkyle, Z représente un atome d'halogène choisi par exemple parmi le chlore et le brome, Z étant de préférence un atome de chlore, x prend une valeur de 1 à 3. Comme exemples de tels composés de formule AlR"ₓZ₃₋ₓ, on peut mentionner le sesquichlorure d'éthylaluminium (Et₃Al₂Cl₃), le dichloroéthylaluminium (EtAlCl₂), le dichloroisobutylaluminium (*i*BuAlCl₂), le chlorodiéthylaluminium (Et₂AlCl) et le triéthylaluminium (AlEt₃). Parmi les aluminoxanes utilisables selon l'invention, on peut citer le méthylaluminoxane et le méthylaluminoxane modifié (MMAO). Ces agents activateurs peuvent être utilisé seuls ou en mélange.

Selon la nature du complexe organometallique [Ti(OR)ₙY₍₄₋ₙ₎], l'agent activateur peut également être choisi dans le groupe des acides de Lewis de type tris(aryl)borane tels que le tris(perfluorophényl)borane, le tris(3,5-bis(trifluorométhyl)phényl)borane, le tris(2,3,4,6-tétrafluorophényl)borane, le tris(perfluoronaphtyl)borane, le tris(perfluobiphényl)borane et leurs dérivés. Il est aussi possible d'utiliser comme activateur un (aryl)borate associé à un cation triphénylcarbénium ou à un cation ammonium trisubstitué tels que le triphénylcarbenium tétrakis(perfluorophényl)borate, le N,N-diméthylanilinium tétrakis(perfluorophényl)borate, le N,N-diéthylanilinium tétrakis(3,5-bis(trifluorométhyl)phényl)borate, le triphénylcarbenium tétrakis(3,5-bis(trifluorométhyl)phényl) borate.

Sans être lié par une quelconque théorie, le groupement fonctionnel L qui se caractérise par la présence d'un hétéroatome choisi parmi l'azote, l'oxygène, le phosphore, le soufre; l'antimoine et l'arsenic ou par la présence d'un groupement aromatique, est susceptible d'interagir avec le centre métallique Ti en formant une liaison par exemple de type dative favorisant ainsi la formation du complexe actif en catalyse et contribuant à sa stabilité. Sans être limitatifs, les exemples ci-dessous illustrent les ligands "O-(CR¹⁰R¹¹)ₙ-X-L" selon l'invention. Les ligands sont représentés ci dessous sous leur forme protonée.

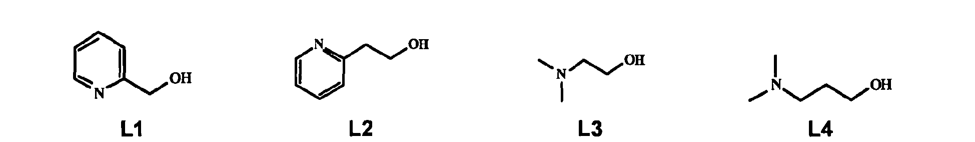

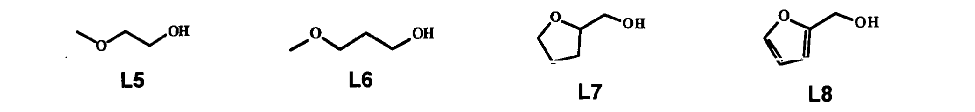

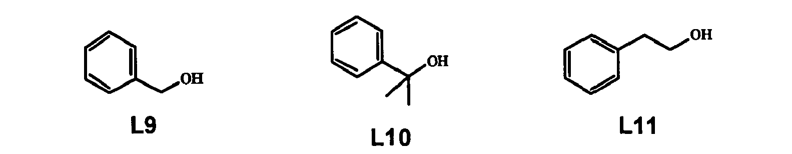

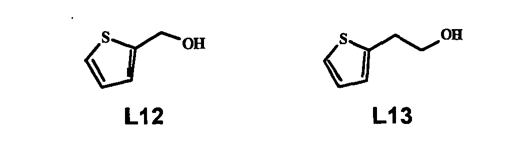

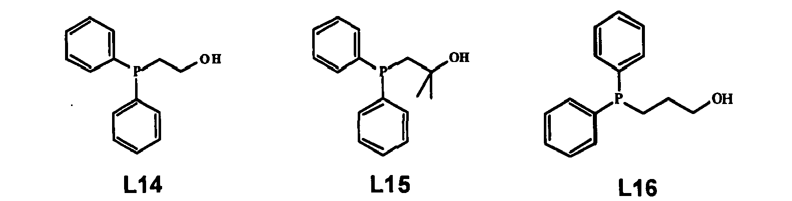

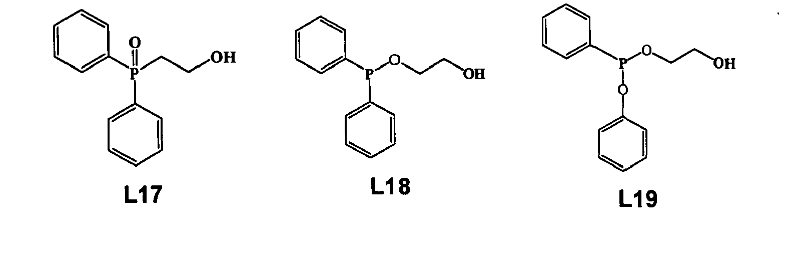

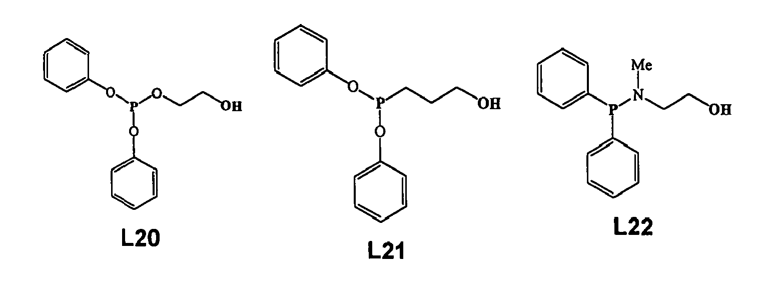

### Procédé de préparation du complexe organométallique

Le procédé de préparation du complexe organométallique du titane de la composition catalytique utilisée dans le procédé selon l'invention, se fait selon les méthodes connues dans la littérature concernant la synthèse des complexes organométalliques comportant au moins un ligand alcoxy. Tout procédé de préparation de ce composé peut convenir, comme par exemple la réaction du ligand de type alcoxy fonctionnalisé par un hétéroatome choisi parmi l'azote, l'oxygène, le phosphore ou le soufre ou par un groupement aromatique, avec un sel de titane directement ou en présence d'un solvant organique, tel que par exemple un éther, un aicane tel que par exemple ie pentane et ie cyclohexane, un solvant aromatique tel que par exemple le toluène, un solvant chloré tel que par exemple le dichlorométhane ou le chlorobenzène.

Selon un mode de réalisation préféré dudit procédé de préparation, le complexe organométallique est préparé *in situ* dans le solvant utilisé pour la réaction de dimérisation. Dans ce cas, l'ordre de mélange du sel de titane n'est pas critique. Cependant, de manière préférée, on prépare d'abord une solution d'un composé de titane soluble en milieu organique et on ajoute ensuite le ligand de type alcoxy fonctionnalisé par un hétéroatome choisi parmi l'azote, l'oxygène, le phosphore ou le soufre ou par un groupement aromatique.

Selon un autre mode de réalisation préféré dudit procédé de préparation, ledit complexe organométallique est isolé avant solubilisation dans le solvant de la réaction de dimérisation.

### Procédé de préparation de la composition catalytique utilisée dans le procédé selon l'invention.

Selon un mode de réalisation préféré du procédé de préparation de ladite composition catalytique et lorsqu'un agent activateur est utilisé, les deux composants de ladite composition catalytique, c'est à dire le complexe organométallique [Ti(OR)ₙY₍₄₋ₙ₎] et l'agent activateur, peuvent être mis en contact dans un ordre quelconque dans un solvant choisi dans le groupe formé par les hydrocarbures aliphatiques et cycloaliphatiques tels que l'hexane, le cyclohexane, l'heptane, le butane ou l'isobutane, par un hydrocarbure insaturé comme une monooléfine ou une dioléfine comportant par exemple de 4 à 20 atomes de carbone, par un hydrocarbure aromatique tel que le benzène, le toluène, l'ortho-xylène, le mésitylène, l'éthylbenzène ou par un hydrocarbure chloré tel que le chlorobenzène ou le dichlorométhane, purs ou en mélange. On utilise avantageusement les hydrocarbures aliphatiques comme le cyclohexane ou le n-heptane et les hydrocarbures aromatiques comme l'ortho-xylène.

Selon un autre mode de réalisation préféré du procédé de préparation de ladite composition catalytique et lorsqu'un agent activateur est utilisé, on ajoute l'agent activateur dans une solution contenant le complexe organométallique du titane.

La concentration du titane dans la solution catalytique est avantageusement comprise entre 1.10⁻⁴ à 1 mole/L, de préférence de entre 1.10⁻³ et 0,5 mole/L.

Le rapport molaire entre l'agent activateur optionnel et le complexe organométallique de titane est avantageusement compris entre 1/1 et 2000/1, de préférence entre 2/1 à 800/1 et de manière préférée entre 2/1 et 500/1.

La température à laquelle les composants du système catalytique sont mélangés est avantageusement comprise entre -10 et +180°C, de préférence entre 0 et +150°C, par exemple à une température voisine de l'ambiante (15 à 30 °C). Le mélange peut être effectué sous une atmosphère d'éthylène ou de gaz inerte.

### Réaction de dimérisation:

Le procédé selon l'invention est un procédé de dimérisation sélective de l'éthylène en butène-1 mettant en oeuvre la composition catalytique décrite ci-dessus.

Selon un mode de réalisation préféré, on utilise le titane comme métal, le triéthylaluminium comme agent activateur et un rapport molaire agent activateur sur titane compris entre 1 et 5 pour la dimérisation de l'éthylène.

La réaction de dimérisation de l'éthylène est avantageusement effectuée sous une pression totale de 0,5 à 15 MPa, de préférence de 1 à 10 MPa, et à une température de 20 à 180°C, de préférence de 40 à 140 C.

Selon un mode de réalisation préféré, la réaction de dimérisation est mise en oeuvre en discontinu. On introduit un volume choisi de la solution catalytique, constituée comme décrit ci-dessus, dans un réacteur muni des dispositifs habituels d'agitation, de chauffage et de refroidissement, puis on pressurise par de l'éthylène à la pression désirée, et on ajuste la température à la valeur souhaitée. Le réacteur de dimérisation est maintenu à pression constante par introduction d'éthylène jusqu'à ce que le volume total de liquide produit représente, par exemple, de 2 à 50 fois le volume de la solution catalytique primitivement introduit. On détruit alors le catalyseur par tout moyen habituel connu de l'homme du métier, puis on soutire et on sépare les produits de la réaction et le solvant.

Selon un autre mode de réalisation préféré, la réaction catalytique de dimérisation est mise en oeuvre en continu. La solution catalytique est injectée en même temps que l'éthylène dans un réacteur agité par les moyens mécaniques classiques connus de l'homme du métier ou par une recirculation extérieure, et maintenu à la température souhaitée. On peut aussi injecter séparément les composants du catalyseur dans le milieu réactionnel. L'éthylène est introduit par une vanne d'admission asservie à la pression, qui maintient celle-ci constante. Le mélange réactionnel est soutiré au moyen d'une vanne asservie au niveau liquide de façon à maintenir celui-ci constant. Le catalyseur est détruit en continu par tout moyen habituel connu de l'homme du métier, puis les produits issus de la réaction ainsi que le solvant sont séparés, par exemple par distillation. L'éthylène qui n'a pas été transformé peut être recyclé dans le réacteur. Les résidus de catalyseur inclus dans une fraction lourde peuvent être incinérés.

### Produits obtenus :

Le procédé selon l'invention permet la production sélective de butène-1. Ce composé trouve une utilisation en tant que comonomères avec l'éthylène dans la fabrication du polyéthylène basse densité linéaire.

### Les exemples suivants illustrent l'invention.

### EXEMPLE 1: Synthèse du complexe [(L7)₂Ti(OiPr)₂]

Dans un schlenk sous argon à température ambiante, on introduit 3,6 g (35 mmol) de ligand L7, 10 mL de cyclohexane sec ainsi que 5 g (17,5 mmol) de [Ti(OiPr)₄]. Ce mélange est ensuite porté à reflux 30min puis agité, toujours sous argon, pendant une nuit. L'évaporation du solvant conduit au complexe [(L7)₂Ti(OiPr)₂] sous forme d'une huile orange. Le rendement est quasi quantitatif. La structure du complexe est confirmée par des analyses RMN ¹H et ¹³C.

### EXEMPLE 2: Synthèse du complexe [(L8)₂Ti(OiPr)₂]

Dans un schlenk sous argon à température ambiante, on introduit 3,4 g (35 mmol) de ligand L8, 10 mL de cyclohexane sec ainsi que 5,0 g (17,5 mmol) de [Ti(OiPr)₄]. Ce mélange est ensuite porté à reflux 30min puis agité, toujours sous argon, pendant une nuit. L'évaporation du solvant conduit au complexe [(L8)₂Ti(OiPr)₂] sous forme d'une huile orange foncée. Le rendement est quasi quantitatif. La structure du complexe est confirmée par des analyses RMN ¹H et ¹³C.

### EXEMPLE 3: Synthèse du complexe [(L9)₂Ti(OiPr)₂]

Dans un schlenk sous argon à température ambiante, on introduit 3,8 g (35 mmol) de ligand L9, 10 mL de cyclohexane sec ainsi que 5,0 g (17,5 mmol) de [Ti(O*i*Pr)₄]. Ce mélange est ensuite porté à reflux 30min puis agité, toujours sous argon, pendant une nuit. L'évaporation du solvant conduit au complexe [(L9)₂Ti(O*i*Pr)₂] sous forme d'une huile incolore. Le rendement est quasi quantitatif. La structure du complexe est confirmée par des analyses RMN ¹H et ¹³C.

### EXEMPLE 4: Synthèse du complexe [(L11)₂Ti(OiPr)₂]

Dans un schlenk sous argon à température ambiante, on introduit 4,3 g (35 mmol) de ligand L11, 10 mL de cyclohexane sec ainsi que 5,0 g (17,5 mmol) de [Ti(O*i*Pr)₄]. Ce mélange est ensuite porté à reflux 30min puis agité, toujours sous argon, pendant une nuit. L'évaporation du solvant conduit au complexe [(L11)₂Ti(O*i*Pr)₂] sous forme d'une huile orange. Le rendement est quasi quantitatif. La structure du complexe est confirmée par des analyses RMN ¹H et ¹³C.

### EXEMPLE 5: Synthèse du complexe [(L12)₂Ti(OiPr)₂]

Dans un schlenk sous argon à température ambiante, on introduit 4,0 g (35 mmol) de ligand L13, 10 mL de cyclohexane sec ainsi que 5,0 g (17,5 mmol) de [Ti(O*i*Pr)₄]. Ce mélange est ensuite porté à reflux 30min puis agité, toujours sous argon, pendant une nuit. L'évaporation du solvant conduit au complexe [(L12)₂Ti(O*i*Pr)₂] sous forme d'un liquide jaune. Le rendement est quasi quantitatif. La structure du complexe est confirmée par des analyses RMN ¹H et ¹³C.

### EXEMPLE 6: Synthèse du complexe [(L14)₂Ti(OiPr)₂]

Dans un schlenk sous argon à température ambiante, on introduit 3,2 g (14 mmol) de ligand L15, 10 mL de cyclohexane sec ainsi que 2,0 g (7 mmol) de [Ti(O*i*Pr)₄]. Ce mélange est ensuite porté à reflux 30min puis agité, toujours sous argon, pendant une nuit. L'évaporation du solvant conduit au complexe [(L14)₂Ti(O*i*Pr)₂] sous forme d'un liquide visqueuse jaune. Le rendement est quasi quantitatif. La structure du complexe est confirmée par des analyses RMN ¹H, ¹³C et ³¹P.

### EXEMPLE 7: Synthèse du complexe [(L16)₂Ti(OiPr)₂]

Dans un schlenk sous argon à température ambiante, on introduit 3,4 g (14 mmol) de ligand L17, 10 mL de cyclohexane sec ainsi que 2,0 g (7 mmol) de [Ti(O*i*Pr)₄]. Ce mélange est ensuite porté à reflux 30min puis agité, toujours sous argon, pendant une nuit. L'évaporation du solvant conduit au complexe [(L16)₂Ti(O*i*Pr)₂] sous forme d'un liquide visqueuse jaune. Le rendement est quasi quantitatif. La structure du complexe est confirmée par des analyses RMN ¹H,¹³C et ³¹P.

### EXEMPLE 8: Synthèse du complexe [(L16)₂Ti(OnBu)₂]

Dans un schlenk sous argon à température ambiante, on introduit 2,9 g (12 mmol) de ligand L15, 10 mL de cyclohexane sec ainsi que 2,0 g (6 mmol) de [Ti(O*n*Bu)₄]. Ce mélange est ensuite porté à reflux 30min puis agité, toujours sous argon, pendant une nuit. L'évaporation du solvant conduit au complexe [(L16)₂Ti(O*n*Bu)₂] sous forme d'un liquide visqueuse jaune. Le rendement est quasi quantitatif. La structure du complexe est confirmée par des analyses RMN ¹H, ¹³C, ³¹P et par analyse élémentaire.

### EXEMPLES 9 à 16 (selon l'invention): Dimérisation sélective de C₂H₄.

Dans un autoclave en acier inoxydable d'un volume utile de 35 mL, muni d'un chauffage électrique et d'un système refroidissement par vortex d'air comprimé permettant de réguler la température, on introduit, dans l'ordre, sous atmosphère d'argon, 0,15 mmol de complexe [(L)ₙTi(O*i*Pr)₄₋ₙ] (ou [(L)ₙTi(OnBu)₄₋ₙ]) tel que décrit dans l'invention préalablement solubilisé dans le cyclohexane. On introduit ensuite 0,45 mmole de triéthylaluminium en solution dans le cyclohexane, soit un rapport molaire Al/Ti = 3. La quantité totale de cyclohexane est de 6 mL. On introduit alors dans l'autoclave de l'éthylène de manière à maintenir une pression constante de 2 MPa. Après un temps "t" de réaction, l'introduction d'éthylène est arrêtée et le réacteur refroidit à température ambiante. L'autoclave est ensuite dépressurisé et le système catalytique neutralisé par injection de 1 ml d'eau. On recueille une fraction gazeuse et une fraction liquide qui sont analysées par chromatographie. Le cas échéant, on récupère également une faible quantité de polyéthylène.
Le tableau 1 suivant reprend de manière détaillée l'ensemble des résultats obtenus:

**Tableau 1: Bilan des essais selon l'invention**

| N° | Nature du complexe | Temps (h) | Productivité (g/gTi/h) | Distribution (%pds) | | |
|---|---|---|---|---|---|---|
| | | | | C4 (α1) | C6 (α2) | PE |
| 9 | [(L7)₂Ti(OiPr)₂] | 1 | 600 | 95 (99+) | 3 (15) | 2 |
| 10 | [(L9)₂Ti(OiPr)₂] | 1 | 700 | 91 (99) | 7,5 (6) | 0,5 |
| 11 | [(L11)₂Ti(OiPr)₂] | 1 | 1400 | 94 (99+) | 5,5 (8) | 0,5 |
| 12 | [(L8)₂Ti(OiPr)₂] | 1 | 800 | 94,5 (99+) | 4,5 (9) | 1 |
| 13 | [(L12)₂Ti(OiPr)₂] | 1 | 500 | 95 (99+) | 4 (11) | 1 |
| 14 | [(L14)₂Ti(OiPr)₂] | 0,43 | 3400 | 94 (99+) | 6 (12) | <0,5 |
| 15 | [(L16)₂Ti(OiPr)₂] | 0,22 | 6600 | 92 (99+) | 8 (12) | <0,5 |
| 16 | [(L16)₂Ti(OnBu)₂] | 0,15 | 9700 | 93 (99+) | 7 (9) | <0,5 |

Dans ce tableau, la productivité est définie comme étant la masse d'éthylène (C₂H₄) consommée par gramme de titane introduit initialement et par heure.

La distribution C4 est la quantité d'oléfines ayant un nombre d'atome de carbone égale à 4 dans la distribution totale.
(α1) représente la sélectivité en butène-1 dans la coupe C4.
   De même, la distribution C6 est la quantité d'oléfines ayant un nombre d'atome de carbone égale à 6 dans la distribution totale.
(α2) représente la sélectivité en hexène-1 dans la coupe C6.
   La sélectivité en butène-1 dans la coupe C4 et en hexène-1 dans la coupe C6 est mesurée par chromatographie en phase gazeuse selon une méthode connue de l'homme du métier.

### EXEMPLE 17 à 20 (comparatif): Dimérisation sélective de C₂H₄ par le [Ti(OiPr)₄] en présence d'additifs organiques non conformes à l'invention.

Les exemples 17 à 20 du tableau 2 ont été réalisés dans les mêmes conditions que celles décrites dans le tableau 1 (le temps de réaction est égal à 1 h). Ces exemples illustrent l'effet négatif des additifs organiques présentant des hétéroatomes mais non conformes à l'invention (et donc l'intérêt du procédé selon l'invention) sur la productivité du [Ti(O*i*Pr)₄] en dimérisation sélective de l'éthylène en butène-1.

**Tableau 2: Bilan des essais comparatifs**

| N° | Nature du complexe | Nature de l'additif externe | Ratio molaire "Additif / Ti" | Productivité (g/gTi/h) | Distribution (%pds) | | |
|---|---|---|---|---|---|---|---|
| | | | | | C4 (α) | C6 (α) | PE |
| 17 | [Ti(OiPr)₄] | THF | 2 | 300 | 97 (99⁺) | 3 (15) | <0,5 |
| 18 | [Ti(OiPr)₄] | Pyridine | 2 | <100 | 99 (99⁺) | <0,5 | <0,5 |
| 19 | [Ti(OiPr)₄] | MeOBu | 2 | 700 | 95 (99+) | <5 | <0,5 |
| 20 | [Ti(OiPr)₄] | PPh₃ | 2 | 1300 | 96 (99+) | 3,5 (13) | 0,5 |

## Revendications

1. Composition catalytique comprenant comprenant au moins un complexe organométallique du titane, ledit complexe organométallique contenant au moins un ligand de type alcoxy fonctionnalisé par un hétéroatome choisi parmi l'azote, l'oxygène, le phosphore, le soufre l'arsenic et l'antimoine ou par un groupement aromatique, et ayant pour formule générale :
[Ti(OR)ₙ(Y)₍₄₋ₙ₎]
dans laquelle :
- Y est un radical hydrocarbyl comprenant de 1 à 30 atomes de carbone ou un radical choisi dans le groupe formé par les halogénures, les alcoxy R'O-, les amido R'₂N- et les carboxylates R'COO-, où R' est un radical hydrocarbyl, de préférence non fonctionnalisé,
comprenant de 1 à 30 atomes de carbone,
- n peut prendre les valeurs entières de 1 à 4,
- Le ligand -OR est un composé organique choisi dans la famille des ligands alcoxy dont la structure générale est proposée ci-après :
O-(CR¹⁰R¹¹)ₙ-X-L
dans laquelle :
- le groupement fonctionnel L est un groupement comprenant un hétéroatome choisi parmi l'azote, l'oxygène, le phosphore, le soufre, l'arsenic et l'antimoine ou un groupement aromatique,
- Le groupement X représente un groupement hydrocarboné (CR⁷R⁸), un atome d'oxygène, ou un groupement comprenant un atome d'azote -NR⁹,
- Les groupements R⁷, R⁸, R⁹, R¹⁰ et R¹¹ représentent un atome d'hydrogène ou une chaîne hydrocarbonée, cyclique ou non, comprenant 1 à 30 atomes de carbone, et comprenant optionnellement un hétéroatome.
- n peut prendre les valeurs entières de 0 à 30.

2. Composition catalytique selon la revendication 1 dans laquelle ledit groupement fonctionnel L est un groupement comprenant un hétéroatome choisi parmi les groupements - NR¹R², -OR³, -PR⁴R⁵, et -SR⁶ dans lesquels les groupements R¹, R², R³, R⁴, R⁵, R⁶ représentent un atome d'hydrogène ou une chaîne hydrocarbonée, cyclique ou non, comprenant 1 à 30 atomes de carbone.

3. Composition catalytique selon l'une des revendications 1 ou 2 dans laquelle ladite composition catalytique comprend un composé d'hydrocarbylaluminium appelé agent activateur, choisi dans le groupe formé par les composés tris(hydrocarbyl)aluminium et les composés chlorés ou bromés d'hydrocarbylaluminium et les aluminoxanes.

4. Composition catalytique selon l'une des revendications 1 à 3 dans laquelle ledit groupement le groupement (CR¹⁰R¹¹)ₙ est choisi parmi les groupements suivants : -CH₂-, - (CH₂)₂-, -(CH₂)₃-, -(CH₂)₄-, -(CH₂)₅-, -C(CH₃)₂-, -C(CH₃)₂-CH₂-, -C(CH₃)₂-CH₂-CH₂-, -C(CF₃)₂-, -C(CF₃)₂-CH₂- et -C(CF₃)₂-CH₂-CH₂-.

5. Composition catalytique selon l'une des revendications 1 à 4 dans laquelle ledit groupement fonctionnel L est choisi parmi les groupements suivants : méthoxy (-OMe), butoxy (-OBu), diméthylamino (-NMe₂), pyrrolidino (C₄H₈N), pyridino (-C₅H₄N), phosphino (-PR₂) dans lequel R est un groupement alkyl ou aryl substitué ou non, thiofène (-C₄H₃S), tétrahydrofurane (-C₄H₇O), furane (-C₄H₃O) et phényl (-C₆H₅), lesdits groupements pouvant être substitués ou non.

6. Composition catalytique selon la revendication 5 dans laquelle ledit groupement L est le groupement phosphino (-PR₂) dans lequel R est un groupement alkyl ou aryl substitué ou non.

7. Composition catalytique selon l'une des revendications 1 à 6 dans laquelle Y est un radical choisi dans le groupe formé par les alcoxy R'O- où R' est un radical hydrocarbyl comprenant de 1 à 30 atomes de carbone.

8. Composition catalytique selon l'une des revendications 1 à 7 dans laquelle X représente un groupement hydrocarboné (CR⁷R⁸).

9. Procédé de dimérisation sélective de l'éthylène en butène-1 mettant en oeuvre une composition catalytique selon l'une des revendications 1 à 8.
